# EUROPEAN PATENT APPLICATION

(11) **EP 0 624 650 A2**
(43) Date of publication of application: **17.11.1994**
(21) Application number: 94302949.6
(22) Date of filing: 25.04.1994
(51) Int. Cl.: C12N 15/31, C12P 21/02, C07K 13/00

(54) **FemB gene of Staphylococcus epidermidis, FemB protein, and vectors and microorganisms comprising the FemB gen**

(30) Priority: 30.04.1993 US 57164
(71) Applicant: ELI LILLY AND COMPANY, Indianapolis, Indiana 46285 (US)
(72) Inventor: Alborn, Jr., William Ernest, Carmel, Indiana 46032 (US); Hoskins, Jo Ann, Indianapolis, Indiana 46256 (US); Skatrud, Paul Luther, Greenwood, Indiana 46143 (US)
(74) Representative: Hudson, Christopher Mark

(57) **Abstract**

The instant invention provides the *femB* gene of *Staphylococcus epidermidis* and all degenerate sequences thereof, the protein encoded by the *femB* gene (FemB), and vectors and microorganisms comprising genes encoding the FemB protein.

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION:
FEMB GENE OF STAPHYLOCOCCUS EPIDERMIDIS, FEMB PROTEIN, AND
VECTORS AND MICROORGANISMS COMPRISING THE FEMB GENE

(iii) NUMBER OF SEQUENCES: 4

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(vi) CURRENT APPLICATION DATA:
(A) APPLICATION NUMBER: 94302949.6

(2) INFORMATION FOR SEQ ID NO:1:

(i) SEQUENCE CHARACTERISTICS:
(A) LENGTH: 1251 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION: 1..1251

(xi) SEQUENCE DESCRIPTION: SEQ ID NO:1:

ATG AAA TTT ACA GAG TTA ACA GTT AAA GAA TTT GAA AAC TTT GTA CAA 48
Met Lys Phe Thr Glu Leu Thr Val Lys Glu Phe Glu Asn Phe Val Gln
1               5                   10                  15

AAT CCA TCA TTA GAA AGT CAT TAT TTC CAA GTG AAG GAA AAT ATT GCT 96
Asn Pro Ser Leu Glu Ser His Tyr Phe Gln Val Lys Glu Asn Ile Ala
            20                  25                  30

ACA CGT GAA TCA GAT GGG TTT CAA GTA GTG TTA TTA GGT GTA AAA GAC 144
Thr Arg Glu Ser Asp Gly Phe Gln Val Val Leu Leu Gly Val Lys Asp
        35                  40                  45

GAC GAC AAT AGA GTG ATA GCA GCT AGC CTG TTT TCT AAA ATC CCT ACA 192
Asp Asp Asn Arg Val Ile Ala Ala Ser Leu Phe Ser Lys Ile Pro Thr
    50                  55                  60

ATG GGC AGT TAT GTG TAT TAT TCC AAT AGA GGC CCT GTA ATG GAC TAT 240
Met Gly Ser Tyr Val Tyr Tyr Ser Asn Arg Gly Pro Val Met Asp Tyr
65                  70                  75                  80

TCA GAT TTA GGT TTA GTG GAT TTT TAT TTA AAA GAG CTT GAT AAA TAT 288
Ser Asp Leu Gly Leu Val Asp Phe Tyr Leu Lys Glu Leu Asp Lys Tyr
                85                  90                  95

TTA CAT CAA CAT CAA TGC TTA TAT GTA AAA TTA GAT CCT TAC TGG TTG 336
Leu His Gln His Gln Cys Leu Tyr Val Lys Leu Asp Pro Tyr Trp Leu
            100                 105                 110

TAT CAA GTT TAT GAT AAA GAT ATT AAT CCT TTA ACA GAA AAA AAT GAT 384
Tyr Gln Val Tyr Asp Lys Asp Ile Asn Pro Leu Thr Glu Lys Asn Asp
        115                 120                 125

GCT TTA GTA AAT CTA TTT AAA TCA CAT GGT TAT GAT CAT CAC GGA TTT 432
Ala Leu Val Asn Leu Phe Lys Ser His Gly Tyr Asp His His Gly Phe
    130                 135                 140

ACA ACC CAA TAT GAT TCT TCC AGC CAA GTT AGA TGG ATG GGG GTA TTA 480
Thr Thr Gln Tyr Asp Ser Ser Ser Gln Val Arg Trp Met Gly Val Leu
145                 150                 155                 160

GAT TTA GAA GGC AAA ACC CCT GCA TCT CTA AGG AAA GAG TTT GAT AGT 528
Asp Leu Glu Gly Lys Thr Pro Ala Ser Leu Arg Lys Glu Phe Asp Ser
                165                 170                 175

CAA AGA AAA CGA AAT ATT AAT AAA GCG ATA AAC TAC GGT GTG AAA GTT 576
Gln Arg Lys Arg Asn Ile Asn Lys Ala Ile Asn Tyr Gly Val Lys Val
            180                 185                 190

AGA TTT CTT AGT AAG GAT GAA TTT GGT TTA TTC TTA GAC TTA TAC CGA 624
Arg Phe Leu Ser Lys Asp Glu Phe Gly Leu Phe Leu Asp Leu Tyr Arg
        195                 200                 205

GAG ACT GAA GCT AGA ACT GGA TTT GCT TCT AAA ACT GAC GAT TAT TTC 672
Glu Thr Glu Ala Arg Thr Gly Phe Ala Ser Lys Thr Asp Asp Tyr Phe
    210                 215                 220

TAT AAC TTT ATA GAG CAT TAT GGC GAT AAA GTA TTA GTT CCT TTA GCT 720
Tyr Asn Phe Ile Glu His Tyr Gly Asp Lys Val Leu Val Pro Leu Ala
225                 230                 235                 240

TAC ATA GAT TTA AAT GAA TAT ATA CAA CAT TTG CAA GAA TCA CTA AAT 768
Tyr Ile Asp Leu Asn Glu Tyr Ile Gln His Leu Gln Glu Ser Leu Asn
                245                 250                 255

GAT AAA GAA AAT CGA CGT GAT GAT ATG ATG GCG AAA GAA AAT AAA ACA 816
Asp Lys Glu Asn Arg Arg Asp Asp Met Met Ala Lys Glu Asn Lys Thr
            260                 265                 270

GAT AAA CAG TTA AAG AAA ATA GCT GAG TTA GAT AAA CAA ATT GAT CAC 864
Asp Lys Gln Leu Lys Lys Ile Ala Glu Leu Asp Lys Gln Ile Asp His
        275                 280                 285

GAT AAA AAA GAA TTG CTT CAA GCT AGT GAA TTA CGT CAA ACA GAT GGC 912
Asp Lys Lys Glu Leu Leu Gln Ala Ser Glu Leu Arg Gln Thr Asp Gly
    290                 295                 300

GAA ATT TTA AAT TTA GCT TCA GGA GTA TAC TTT GCT AAT GCA TAT GAA 960
Glu Ile Leu Asn Leu Ala Ser Gly Val Tyr Phe Ala Asn Ala Tyr Glu
305                 310                 315                 320

GTG AAC TAT TTC TCT GGA GGG TCT TCA GAA AAA TAT AAT CAA TAT ATG 1008
Val Asn Tyr Phe Ser Gly Gly Ser Ser Glu Lys Tyr Asn Gln Tyr Met
                325                 330                 335

GGA CCA TAT GCA ATG CAT TGG CAC ATG ATT AAT TAT TGT TTT GAT AAC 1056
Gly Pro Tyr Ala Met His Trp His Met Ile Asn Tyr Cys Phe Asp Asn
            340                 345                 350

GGT TAT GAT AGA TAT AAT TTC TAT GGC TTA TCA GGT GAT TTT ACT GAA 1104
Gly Tyr Asp Arg Tyr Asn Phe Tyr Gly Leu Ser Gly Asp Phe Thr Glu
        355                 360                 365

AAC AGT GAA GAC TAT GGT GTT TAT CGC TTT AAG AGA GGT TTT AAT GTT 1152
Asn Ser Glu Asp Tyr Gly Val Tyr Arg Phe Lys Arg Gly Phe Asn Val
    370                 375                 380

AGG ATT GAG GAA TTA ATC GGT GAT TTC TAT AAA CCA ATC AAT AAA GTG 1200
Arg Ile Glu Glu Leu Ile Gly Asp Phe Tyr Lys Pro Ile Asn Lys Val
385                 390                 395                 400

AAA TAT TGG TTA TTC AAT ACA TTA GAT CGC ATA CGT AAT AAA TTG AAA 1248
Lys Tyr Trp Leu Phe Asn Thr Leu Asp Arg Ile Arg Asn Lys Leu Lys
                405                 410                 415

AAA 1251
Lys

(2) INFORMATION FOR SEQ ID NO:2:

      (i) SEQUENCE CHARACTERISTICS:
            (A) LENGTH: 417 amino acids
            (B) TYPE: amino acid
            (D) TOPOLOGY: linear

      (ii) MOLECULE TYPE: protein

      (xi) SEQUENCE DESCRIPTION: SEQ ID NO:2:

Met Lys Phe Thr Glu Leu Thr Val Lys Glu Phe Glu Asn Phe Val Gln
1               5                   10                  15

Asn Pro Ser Leu Glu Ser His Tyr Phe Gln Val Lys Glu Asn Ile Ala
            20                  25                  30

Thr Arg Glu Ser Asp Gly Phe Gln Val Val Leu Leu Gly Val Lys Asp
        35                  40                  45

Asp Asp Asn Arg Val Ile Ala Ala Ser Leu Phe Ser Lys Ile Pro Thr
    50                  55                  60

Met Gly Ser Tyr Val Tyr Tyr Ser Asn Arg Gly Pro Val Met Asp Tyr
65                  70                  75                  80

Ser Asp Leu Gly Leu Val Asp Phe Tyr Leu Lys Glu Leu Asp Lys Tyr
                85                  90                  95
Leu His Gln His Gln Cys Leu Tyr Val Lys Leu Asp Pro Tyr Trp Leu
            100                 105                 110
Tyr Gln Val Tyr Asp Lys Asp Ile Asn Pro Leu Thr Glu Lys Asn Asp
        115                 120                 125
Ala Leu Val Asn Leu Phe Lys Ser His Gly Tyr Asp His His Gly Phe
    130                 135                 140
Thr Thr Gln Tyr Asp Ser Ser Ser Gln Val Arg Trp Met Gly Val Leu
145                 150                 155                 160
Asp Leu Glu Gly Lys Thr Pro Ala Ser Leu Arg Lys Glu Phe Asp Ser
                165                 170                 175
Gln Arg Lys Arg Asn Ile Asn Lys Ala Ile Asn Tyr Gly Val Lys Val
            180                 185                 190
Arg Phe Leu Ser Lys Asp Glu Phe Gly Leu Phe Leu Asp Leu Tyr Arg
        195                 200                 205
Glu Thr Glu Ala Arg Thr Gly Phe Ala Ser Lys Thr Asp Asp Tyr Phe
    210                 215                 220
Tyr Asn Phe Ile Glu His Tyr Gly Asp Lys Val Leu Val Pro Leu Ala
225                 230                 235                 240
Tyr Ile Asp Leu Asn Glu Tyr Ile Gln His Leu Gln Glu Ser Leu Asn
                245                 250                 255
Asp Lys Glu Asn Arg Arg Asp Asp Met Met Ala Lys Glu Asn Lys Thr
            260                 265                 270
Asp Lys Gln Leu Lys Lys Ile Ala Glu Leu Asp Lys Gln Ile Asp His
        275                 280                 285
Asp Lys Lys Glu Leu Leu Gln Ala Ser Glu Leu Arg Gln Thr Asp Gly
    290                 295                 300
Glu Ile Leu Asn Leu Ala Ser Gly Val Tyr Phe Ala Asn Ala Tyr Glu
305                 310                 315                 320
Val Asn Tyr Phe Ser Gly Gly Ser Ser Glu Lys Tyr Asn Gln Tyr Met
                325                 330                 335
Gly Pro Tyr Ala Met His Trp His Met Ile Asn Tyr Cys Phe Asp Asn
            340                 345                 350

Gly Tyr Asp Arg Tyr Asn Phe Tyr Gly Leu Ser Gly Asp Phe Thr Glu
        355                 360                 365

Asn Ser Glu Asp Tyr Gly Val Tyr Arg Phe Lys Arg Gly Phe Asn Val
    370                 375                 380

Arg Ile Glu Glu Leu Ile Gly Asp Phe Tyr Lys Pro Ile Asn Lys Val
385                 390                 395                 400

Lys Tyr Trp Leu Phe Asn Thr Leu Asp Arg Ile Arg Asn Lys Leu Lys
                405                 410                 415

Lys

(2) INFORMATION FOR SEQ ID NO:3:

(i) SEQUENCE CHARACTERISTICS:
(A) LENGTH: 30 base pairs
(B) TYPE: nucleic acid
(C) STRANDEDNESS: single
(D) TOPOLOGY: linear

(xi) SEQUENCE DESCRIPTION: SEQ ID NO:3:

GAAGGGAATT ATCTAGATAT GAAATTTACA 30

(2) INFORMATION FOR SEQ ID NO:4:

(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:4:

ATCATTTATT TTTACGAATT CAATTTATTA CGTATG 36

## Description

Clinical isolates of staphylococci *(Staphylococcus aureus* and *S.* *epidermidis)* which cause serious infections due to their intrinsic resistance to beta-lactamase-stable beta-lactam antibiotics (*e.g*., methicillin) carry the *mecA* gene. Song et al., *FEBS Lett.* **221**:167-171 (1987). This gene encodes a putative cell wall biosynthetic enzyme referred to as penicillin binding protein 2a (PBP2a). PBP2a, which binds beta-lactams only at concentrations well above therapeutic efficacy, apparently can functionally substitute for all the staphylococcal PBPs and permit growth when the host organism is threatened by beta-lactams. Hartman and Tomasz, *J. Bacteriol.* **158**:513-516 (1984). Wu et al., *Antimicrob. Agents Chemother.* **36**:533-539 (1992) and Ryffel et al., *Gene* **94**:137-138 (1990).

The *mecA* gene is not a normal part of the staphylococcal genome. The organism which donated *mecA* to the staphylococci remains unidentified. Despite the uniform presence of *mecA* in methicillin-resistant clinical isolates, these isolates vary considerably in their degree of resistance to methicillin. This variation in phenotypic expression within a population has been referred to as heterogenous expression. Matthews and Stewart, *FEMS Microbiol. Lett.* **22**:161-166 (1984). Typically, most cells exhibit low-level resistance to methicillin and only a minority of the population express high-level resistance, perhaps only one in 10⁸ cells. Tomasz et al., *Antimicrob*. *Agents Chemother.* **35**:124-129 (1991). Although expression of methicillin resistance is dependent upon the presence of PBP2a, it appears to be somewhat independent of the amount of PBP2a, suggesting important roles for other factors. Chambers and Hackbarth, *Antimicrob. Agents Chemother.* **31**:1982-1988 (1987) and Murakami and Tomasz, *J. Bacteriol.* **171**:874-879 (1989).

*Tn551* insertional mutagenesis of methicillin-resistant *S*. *aureus* revealed numerous sites which influence the level of methicillin resistance but are not linked to *mecA* and do not perturb the expression of PBP2a. Berger-Bächi et al., *Antimicrob. Agents Chemother.* **36**:1367-1373 (1992); Kornblum et al., *Eur. J. Clin. Microbiol.* **5**:714-718 (1986); Berger-Bachi et al., *Mol. Gen. Genet.* **219**:263-269 (1989) and Maidhof et al., *J. Bacteriol.* **173**:3507-3513 (1991). Those factors described thus far generally depress the MIC of beta-lactam resistant strains. Some of the genetic loci which demonstrate such an effect on methicillin resistance were designated factors essential for methicillin resistance *(fem).* Berger-Bächi et al., *Mol. Gen. Genet.* **219**:263-269 (1989). In contrast to *mecA,* the genes which encode influential factors are probably present in both resistant and susceptible strains of *S*. *aureus* and *S*. *epidermidis.* Information obtained from gene disruption studies of *femA* and *femB* in *S*. *aureus* indicated that in addition to enhanced sensitivity to methicillin, homogeneously methicillin-resistant *S*. *aureus* strains carrying such gene disruptions have a reduced glycine content in the peptidoglycan component of their cell walls (Maidhof et al., *J. Bacteriol.* **173**:3507-3513 (1991)) and exhibit reduced rates of cell wall turnover and autolysis (de Jonge et al., *J. Bacteriol.* **173**:1105-1110 (1991)).

Genetic factors, other than *mecA,* that influence the expression of methicillin resistance in *S*. *epidermidis* have, until now, not been described at the molecular level. The present invention provides DNA sequences encoding the FemB protein of *Staphylococcus epidermidis,* the FemB protein itself, and vectors and microorganisms comprising the *femB* gene of *S*. *epidermidis.*

The present invention provides DNA sequences encoding the FemB protein of *Staphylococcus epidermidis,* including the natural gene sequence designated *femB* (SEQ ID NO:1). Thus, included in the present invention is any DNA compound that comprises an isolated DNA sequence encoding SEQ ID NO:2. SEQ ID NO:2 is as follows:

Met Lys Phe Thr Glu Leu Thr Val Lys Glu Phe Glu Asn Phe Val Gln
1               5                   10                  15

Asn Pro Ser Leu Glu Ser His Tyr Phe Gln Val Lys Glu Asn Ile Ala
            20                  25                  30

Thr Arg Glu Ser Asp Gly Phe Gln Val Val Leu Leu Gly Val Lys Asp
        35                  40                  45

Asp Asp Asn Arg Val Ile Ala Ala Ser Leu Phe Ser Lys Ile Pro Thr
    50                  55                  60

Met Gly Ser Tyr Val Tyr Tyr Ser Asn Arg Gly Pro Val Met Asp Tyr
65                  70                  75                  80

Ser Asp Leu Gly Leu Val Asp Phe Tyr Leu Lys Glu Leu Asp Lys Tyr
                85                  90                  95

Leu His Gln His Gln Cys Leu Tyr Val Lys Leu Asp Pro Tyr Trp Leu
            100                 105                 110

Tyr Gln Val Tyr Asp Lys Asp Ile Asn Pro Leu Thr Glu Lys Asn Asp
        115                 120                 125

Ala Leu Val Asn Leu Phe Lys Ser His Gly Tyr Asp His His Gly Phe
    130                 135                 140

Thr Thr Gln Tyr Asp Ser Ser Ser Gln Val Arg Trp Met Gly Val Leu
145                 150                 155                 160

Asp Leu Glu Gly Lys Thr Pro Ala Ser Leu Arg Lys Glu Phe Asp Ser
                165                 170                 175

Gln Arg Lys Arg Asn Ile Asn Lys Ala Ile Asn Tyr Gly Val Lys Val
            180                 185                 190

Arg Phe Leu Ser Lys Asp Glu Phe Gly Leu Phe Leu Asp Leu Tyr Arg
        195                 200                 205

Glu Thr Glu Ala Arg Thr Gly Phe Ala Ser Lys Thr Asp Asp Tyr Phe
    210                 215                 220

Tyr Asn Phe Ile Glu His Tyr Gly Asp Lys Val Leu Val Pro Leu Ala
225                 230                 235                 240

Tyr Ile Asp Leu Asn Glu Tyr Ile Gln His Leu Gln Glu Ser Leu Asn
                245                 250                 255

Asp Lys Glu Asn Arg Arg Asp Asp Met Met Ala Lys Glu Asn Lys Thr
            260                 265                 270

Asp Lys Gln Leu Lys Lys Ile Ala Glu Leu Asp Lys Gln Ile Asp His
        275                 280                 285

Asp Lys Lys Glu Leu Leu Gln Ala Ser Glu Leu Arg Gln Thr Asp Gly
    290                 295                 300

Glu Ile Leu Asn Leu Ala Ser Gly Val Tyr Phe Ala Asn Ala Tyr Glu
305                 310                 315                 320

Val Asn Tyr Phe Ser Gly Gly Ser Ser Glu Lys Tyr Asn Gln Tyr Met
                325                 330                 335

Gly Pro Tyr Ala Met His Trp His Met Ile Asn Tyr Cys Phe Asp Asn
            340                 345                 350

Gly Tyr Asp Arg Tyr Asn Phe Tyr Gly Leu Ser Gly Asp Phe Thr Glu
        355                 360                 365

Asn Ser Glu Asp Tyr Gly Val Tyr Arg Phe Lys Arg Gly Phe Asn Val
    370                 375                 380

Arg Ile Glu Glu Leu Ile Gly Asp Phe Tyr Lys Pro Ile Asn Lys Val
385                 390                 395                 400

Lys Tyr Trp Leu Phe Asn Thr Leu Asp Arg Ile Arg Asn Lys Leu Lys
                405                 410                 415

Lys

The natural *femB* sequence is encompassed by the present invention as a DNA compound which comprises the isolated DNA sequence which is SEQ ID NO:1. SEQ ID NO:1 is as follows:

ATG AAA TTT ACA GAG TTA ACA GTT AAA GAA TTT GAA AAC TTT GTA CAA 48
Met Lys Phe Thr Glu Leu Thr Val Lys Glu Phe Glu Asn Phe Val Gln
1               5                   10                  15

AAT CCA TCA TTA GAA AGT CAT TAT TTC CAA GTG AAG GAA AAT ATT GCT 96
Asn Pro Ser Leu Glu Ser His Tyr Phe Gln Val Lys Glu Asn Ile Ala
            20                  25                  30

ACA CGT GAA TCA GAT GGG TTT CAA GTA GTG TTA TTA GGT GTA AAA GAC 144
Thr Arg Glu Ser Asp Gly Phe Gln Val Val Leu Leu Gly Val Lys Asp
        35                  40                  45

GAC GAC AAT AGA GTG ATA GCA GCT AGC CTG TTT TCT AAA ATC CCT ACA 192
Asp Asp Asn Arg Val Ile Ala Ala Ser Leu Phe Ser Lys Ile Pro Thr
    50                  55                  60

ATG GGC AGT TAT GTG TAT TAT TCC AAT AGA GGC CCT GTA ATG GAC TAT 240
Met Gly Ser Tyr Val Tyr Tyr Ser Asn Arg Gly Pro Val Met Asp Tyr
65                  70                  75                  80

TCA GAT TTA GGT TTA GTG GAT TTT TAT TTA AAA GAG CTT GAT AAA TAT 288
Ser Asp Leu Gly Leu Val Asp Phe Tyr Leu Lys Glu Leu Asp Lys Tyr
                85                  90                  95

TTA CAT CAA CAT CAA TGC TTA TAT GTA AAA TTA GAT CCT TAC TGG TTG 336
Leu His Gln His Gln Cys Leu Tyr Val Lys Leu Asp Pro Tyr Trp Leu
            100                 105                 110

TAT CAA GTT TAT GAT AAA GAT ATT AAT CCT TTA ACA GAA AAA AAT GAT 384
Tyr Gln Val Tyr Asp Lys Asp Ile Asn Pro Leu Thr Glu Lys Asn Asp
        115                 120                 125

GCT TTA GTA AAT CTA TTT AAA TCA CAT GGT TAT GAT CAT CAC GGA TTT 432
Ala Leu Val Asn Leu Phe Lys Ser His Gly Tyr Asp His His Gly Phe
    130                 135                 140

ACA ACC CAA TAT GAT TCT TCC AGC CAA GTT AGA TGG ATG GGG GTA TTA 480
Thr Thr Gln Tyr Asp Ser Ser Ser Gln Val Arg Trp Met Gly Val Leu
145                 150                 155                 160

GAT TTA GAA GGC AAA ACC CCT GCA TCT CTA AGG AAA GAG TTT GAT AGT 528
Asp Leu Glu Gly Lys Thr Pro Ala Ser Leu Arg Lys Glu Phe Asp Ser
                165                 170                 175

CAA AGA AAA CGA AAT ATT AAT AAA GCG ATA AAC TAC GGT GTG AAA GTT 576
Gln Arg Lys Arg Asn Ile Asn Lys Ala Ile Asn Tyr Gly Val Lys Val
            180                 185                 190

AGA TTT CTT AGT AAG GAT GAA TTT GGT TTA TTC TTA GAC TTA TAC CGA 624
Arg Phe Leu Ser Lys Asp Glu Phe Gly Leu Phe Leu Asp Leu Tyr Arg
        195                 200                 205

GAG ACT GAA GCT AGA ACT GGA TTT GCT TCT AAA ACT GAC GAT TAT TTC 672
Glu Thr Glu Ala Arg Thr Gly Phe Ala Ser Lys Thr Asp Asp Tyr Phe
    210                 215                 220

TAT AAC TTT ATA GAG CAT TAT GGC GAT AAA GTA TTA GTT CCT TTA GCT 720
Tyr Asn Phe Ile Glu His Tyr Gly Asp Lys Val Leu Val Pro Leu Ala
225                 230                 235                 240

TAC ATA GAT TTA AAT GAA TAT ATA CAA CAT TTG CAA GAA TCA CTA AAT 768
Tyr Ile Asp Leu Asn Glu Tyr Ile Gln His Leu Gln Glu Ser Leu Asn
                245                 250                 255

GAT AAA GAA AAT CGA CGT GAT GAT ATG ATG GCG AAA GAA AAT AAA ACA 816
Asp Lys Glu Asn Arg Arg Asp Asp Met Met Ala Lys Glu Asn Lys Thr
            260                 265                 270

GAT AAA CAG TTA AAG AAA ATA GCT GAG TTA GAT AAA CAA ATT GAT CAC 864
Asp Lys Gln Leu Lys Lys Ile Ala Glu Leu Asp Lys Gln Ile Asp His
        275                 280                 285

GAT AAA AAA GAA TTG CTT CAA GCT AGT GAA TTA CGT CAA ACA GAT GGC 912
Asp Lys Lys Glu Leu Leu Gln Ala Ser Glu Leu Arg Gln Thr Asp Gly
    290                 295                 300

GAA ATT TTA AAT TTA GCT TCA GGA GTA TAC TTT GCT AAT GCA TAT GAA 960
Glu Ile Leu Asn Leu Ala Ser Gly Val Tyr Phe Ala Asn Ala Tyr Glu
305                 310                 315                 320

GTG AAC TAT TTC TCT GGA GGG TCT TCA GAA AAA TAT AAT CAA TAT ATG 1008
Val Asn Tyr Phe Ser Gly Gly Ser Ser Glu Lys Tyr Asn Gln Tyr Met
                325                 330                 335

GGA CCA TAT GCA ATG CAT TGG CAC ATG ATT AAT TAT TGT TTT GAT AAC 1056
Gly Pro Tyr Ala Met His Trp His Met Ile Asn Tyr Cys Phe Asp Asn
            340                 345                 350

GGT TAT GAT AGA TAT AAT TTC TAT GGC TTA TCA GGT GAT TTT ACT GAA 1104
Gly Tyr Asp Arg Tyr Asn Phe Tyr Gly Leu Ser Gly Asp Phe Thr Glu
        355                 360                 365

AAC AGT GAA GAC TAT GGT GTT TAT CGC TTT AAG AGA GGT TTT AAT GTT 1152
Asn Ser Glu Asp Tyr Gly Val Tyr Arg Phe Lys Arg Gly Phe Asn Val
    370                 375                 380

AGG ATT GAG GAA TTA ATC GGT GAT TTC TAT AAA CCA ATC AAT AAA GTG 1200
Arg Ile Glu Glu Leu Ile Gly Asp Phe Tyr Lys Pro Ile Asn Lys Val
385                 390                 395                 400

AAA TAT TGG TTA TTC AAT ACA TTA GAT CGC ATA CGT AAT AAA TTG AAA 1248
Lys Tyr Trp Leu Phe Asn Thr Leu Asp Arg Ile Arg Asn Lys Leu Lys
                405                 410                 415

AAA 1251
Lys

The present invention also includes the protein encoded by SEQ ID NO: 1 in purified form. Also included are recombinant DNA vectors, including expression vectors, that comprise DNA sequences encoding FemB.

The restriction site and function maps presented in the accompanying drawings are approximate representations of the recombinant DNA vectors discussed herein. The restriction site information is not exhaustive; therefore, there may be more restriction sites of a given type on the vector than actually shown on the map.

Figure 1 - A restriction site and function map of plasmid pPSJ203.

Figure 2 - A restriction site and function map of plasmid pET-11A.

The instant invention provides the *femB* gene of *Staphylococcus epidermidis* and all degenerate sequences thereof, the protein encoded by the *femB* gene (FemB), and vectors and microorganisms comprising genes encoding the FemB protein. In the practice of the invention as exemplified herein, the FemB protein comprises the amino acid sequence, which is SEQ ID NO 2:

Met Lys Phe Thr Glu Leu Thr Val Lys Glu Phe Glu Asn Phe Val Gln
1               5                   10                  15

Asn Pro Ser Leu Glu Ser His Tyr Phe Gln Val Lys Glu Asn Ile Ala
            20                  25                  30

Thr Arg Glu Ser Asp Gly Phe Gln Val Val Leu Leu Gly Val Lys Asp
        35                  40                  45

Asp Asp Asn Arg Val Ile Ala Ala Ser Leu Phe Ser Lys Ile Pro Thr
    50                  55                  60

Met Gly Ser Tyr Val Tyr Tyr Ser Asn Arg Gly Pro Val Met Asp Tyr
65                  70                  75                  80

Ser Asp Leu Gly Leu Val Asp Phe Tyr Leu Lys Glu Leu Asp Lys Tyr
                85                  90                  95

Leu His Gln His Gln Cys Leu Tyr Val Lys Leu Asp Pro Tyr Trp Leu
            100                 105                 110

Tyr Gln Val Tyr Asp Lys Asp Ile Asn Pro Leu Thr Glu Lys Asn Asp
        115                 120                 125

Ala Leu Val Asn Leu Phe Lys Ser His Gly Tyr Asp His His Gly Phe
    130                 135                 140

Thr Thr Gln Tyr Asp Ser Ser Ser Gln Val Arg Trp Met Gly Val Leu
145                 150                 155                 160

Asp Leu Glu Gly Lys Thr Pro Ala Ser Leu Arg Lys Glu Phe Asp Ser
                165                 170                 175

Gln Arg Lys Arg Asn Ile Asn Lys Ala Ile Asn Tyr Gly Val Lys Val
            180                 185                 190

Arg Phe Leu Ser Lys Asp Glu Phe Gly Leu Phe Leu Asp Leu Tyr Arg
        195                 200                 205

Glu Thr Glu Ala Arg Thr Gly Phe Ala Ser Lys Thr Asp Asp Tyr Phe
    210                 215                 220

Tyr Asn Phe Ile Glu His Tyr Gly Asp Lys Val Leu Val Pro Leu Ala
225                 230                 235                 240

Tyr Ile Asp Leu Asn Glu Tyr Ile Gln His Leu Gln Glu Ser Leu Asn
                245                 250                 255

Asp Lys Glu Asn Arg Arg Asp Asp Met Met Ala Lys Glu Asn Lys Thr
            260                 265                 270

Asp Lys Gln Leu Lys Lys Ile Ala Glu Leu Asp Lys Gln Ile Asp His
        275                 280                 285

Asp Lys Lys Glu Leu Leu Gln Ala Ser Glu Leu Arg Gln Thr Asp Gly
    290                 295                 300

Glu Ile Leu Asn Leu Ala Ser Gly Val Tyr Phe Ala Asn Ala Tyr Glu
305                 310                 315                 320

Val Asn Tyr Phe Ser Gly Gly Ser Ser Glu Lys Tyr Asn Gln Tyr Met
                325                 330                 335

Gly Pro Tyr Ala Met His Trp His Met Ile Asn Tyr Cys Phe Asp Asn
            340                 345                 350

Gly Tyr Asp Arg Tyr Asn Phe Tyr Gly Leu Ser Gly Asp Phe Thr Glu
        355                 360                 365

Asn Ser Glu Asp Tyr Gly Val Tyr Arg Phe Lys Arg Gly Phe Asn Val
    370                 375                 380

Arg Ile Glu Glu Leu Ile Gly Asp Phe Tyr Lys Pro Ile Asn Lys Val
385                 390                 395                 400

Lys Tyr Trp Leu Phe Asn Thr Leu Asp Arg Ile Arg Asn Lys Leu Lys
                405                 410                 415

Lys

The present invention also provides the natural *femB* gene found in *Staphylococcus epidermidis,* embodied in SEQ ID NO: 1: 
ATG AAA TTT ACA GAG TTA ACA GTT AAA GAA TTT GAA AAC TTT GTA CAA 48
Met Lys Phe Thr Glu Leu Thr Val Lys Glu Phe Glu Asn Phe Val Gln
1               5                   10                  15

AAT CCA TCA TTA GAA AGT CAT TAT TTC CAA GTG AAG GAA AAT ATT GCT 96
Asn Pro Ser Leu Glu Ser His Tyr Phe Gln Val Lys Glu Asn Ile Ala
            20                  25                  30

ACA CGT GAA TCA GAT GGG TTT CAA GTA GTG TTA TTA GGT GTA AAA GAC 144
Thr Arg Glu Ser Asp Gly Phe Gln Val Val Leu Leu Gly Val Lys Asp
        35                  40                  45

GAC GAC AAT AGA GTG ATA GCA GCT AGC CTG TTT TCT AAA ATC CCT ACA 192
Asp Asp Asn Arg Val Ile Ala Ala Ser Leu Phe Ser Lys Ile Pro Thr
    50                  55                  60
 
ATG GGC AGT TAT GTG TAT TAT TCC AAT AGA GGC CCT GTA ATG GAC TAT 240
Met Gly Ser Tyr Val Tyr Tyr Ser Asn Arg Gly Pro Val Met Asp Tyr
65                  70                  75                  80

TCA GAT TTA GGT TTA GTG GAT TTT TAT TTA AAA GAG CTT GAT AAA TAT 288
Ser Asp Leu Gly Leu Val Asp Phe Tyr Leu Lys Glu Leu Asp Lys Tyr
                85                  90                  95

TTA CAT CAA CAT CAA TGC TTA TAT GTA AAA TTA GAT CCT TAC TGG TTG 336
Leu His Gln His Gln Cys Leu Tyr Val Lys Leu Asp Pro Tyr Trp Leu
            100                 105                 110

TAT CAA GTT TAT GAT AAA GAT ATT AAT CCT TTA ACA GAA AAA AAT GAT 384
Tyr Gln Val Tyr Asp Lys Asp Ile Asn Pro Leu Thr Glu Lys Asn Asp
        115                 120                 125

GCT TTA GTA AAT CTA TTT AAA TCA CAT GGT TAT GAT CAT CAC GGA TTT 432
Ala Leu Val Asn Leu Phe Lys Ser His Gly Tyr Asp His His Gly Phe
    130                 135                 140

ACA ACC CAA TAT GAT TCT TCC AGC CAA GTT AGA TGG ATG GGG GTA TTA 480
Thr Thr Gln Tyr Asp Ser Ser Ser Gln Val Arg Trp Met Gly Val Leu
145                 150                 155                 160

GAT TTA GAA GGC AAA ACC CCT GCA TCT CTA AGG AAA GAG TTT GAT AGT 528
Asp Leu Glu Gly Lys Thr Pro Ala Ser Leu Arg Lys Glu Phe Asp Ser
                165                 170                 175

CAA AGA AAA CGA AAT ATT AAT AAA GCG ATA AAC TAC GGT GTG AAA GTT 576
Gln Arg Lys Arg Asn Ile Asn Lys Ala Ile Asn Tyr Gly Val Lys Val
            180                 185                 190

AGA TTT CTT AGT AAG GAT GAA TTT GGT TTA TTC TTA GAC TTA TAC CGA 624
Arg Phe Leu Ser Lys Asp Glu Phe Gly Leu Phe Leu Asp Leu Tyr Arg
        195                 200                 205

GAG ACT GAA GCT AGA ACT GGA TTT GCT TCT AAA ACT GAC GAT TAT TTC 672
Glu Thr Glu Ala Arg Thr Gly Phe Ala Ser Lys Thr Asp Asp Tyr Phe
    210                 215                 220

TAT AAC TTT ATA GAG CAT TAT GGC GAT AAA GTA TTA GTT CCT TTA GCT 720
Tyr Asn Phe Ile Glu His Tyr Gly Asp Lys Val Leu Val Pro Leu Ala
225                 230                 235                 240

TAC ATA GAT TTA AAT GAA TAT ATA CAA CAT TTG CAA GAA TCA CTA AAT 768
Tyr Ile Asp Leu Asn Glu Tyr Ile Gln His Leu Gln Glu Ser Leu Asn
                245                 250                 255

GAT AAA GAA AAT CGA CGT GAT GAT ATG ATG GCG AAA GAA AAT AAA ACA 816
Asp Lys Glu Asn Arg Arg Asp Asp Met Met Ala Lys Glu Asn Lys Thr
            260                 265                 270
 
GAT AAA CAG TTA AAG AAA ATA GCT GAG TTA GAT AAA CAA ATT GAT CAC 864
Asp Lys Gln Leu Lys Lys Ile Ala Glu Leu Asp Lys Gln Ile Asp His
        275                 280                 285

GAT AAA AAA GAA TTG CTT CAA GCT AGT GAA TTA CGT CAA ACA GAT. GGC 912
Asp Lys Lys Glu Leu Leu Gln Ala Ser Glu Leu Arg Gln Thr Asp Gly
    290                 295                 300

GAA ATT TTA AAT TTA GCT TCA GGA GTA TAC TTT GCT AAT GCA TAT GAA 960
Glu Ile Leu Asn Leu Ala Ser Gly Val Tyr Phe Ala Asn Ala Tyr Glu
305                 310                 315                 320

GTG AAC TAT TTC TCT GGA GGG TCT TCA GAA AAA TAT AAT CAA TAT ATG 1008
Val Asn Tyr Phe Ser Gly Gly Ser Ser Glu Lys Tyr Asn Gln Tyr Met
                325                 330                 335

GGA CCA TAT GCA ATG CAT TGG CAC ATG ATT AAT TAT TGT TTT GAT AAC 1056
Gly Pro Tyr Ala Met His Trp His Met Ile Asn Tyr Cys Phe Asp Asn
            340                 345                 350

GGT TAT GAT AGA TAT AAT TTC TAT GGC TTA TCA GGT GAT TTT ACT GAA 1104
Gly Tyr Asp Arg Tyr Asn Phe Tyr Gly Leu Ser Gly Asp Phe Thr Glu
        355                 360                 365

AAC AGT GAA GAC TAT GGT GTT TAT CGC TTT AAG AGA GGT TTT AAT GTT 1152
Asn Ser Glu Asp Tyr Gly Val Tyr Arg Phe Lys Arg Gly Phe Asn Val
    370                 375                 380

AGG ATT GAG GAA TTA ATC GGT GAT TTC TAT AAA CCA ATC AAT AAA GTG 1200
Arg Ile Glu Glu Leu Ile Gly Asp Phe Tyr Lys Pro Ile Asn Lys Val
385                 390                 395                 400

AAA TAT TGG TTA TTC AAT ACA TTA GAT CGC ATA CGT AAT AAA TTG AAA 1248
Lys Tyr Trp Leu Phe Asn Thr Leu Asp Arg Ile Arg Asn Lys Leu Lys
                405                 410                 415

AAA 1251
Lys
The synthesis of the FemB protein of the present invention may proceed by solid phase peptide synthesis or by recombinant methods. Both methods are described in U.S. Patent No. 4,617,149, the entire teaching of which is herein incorporated by reference. Recombinant methods are preferred if a high yield is desired. The principles of solid phase chemical synthesis of polypeptides are well known in the art and may be found in general texts in the area such as Dugas, H. and Penney, C., *Bioorganic Chemistry* (1981), Springer-Verlag, New York, pgs. 54-92.

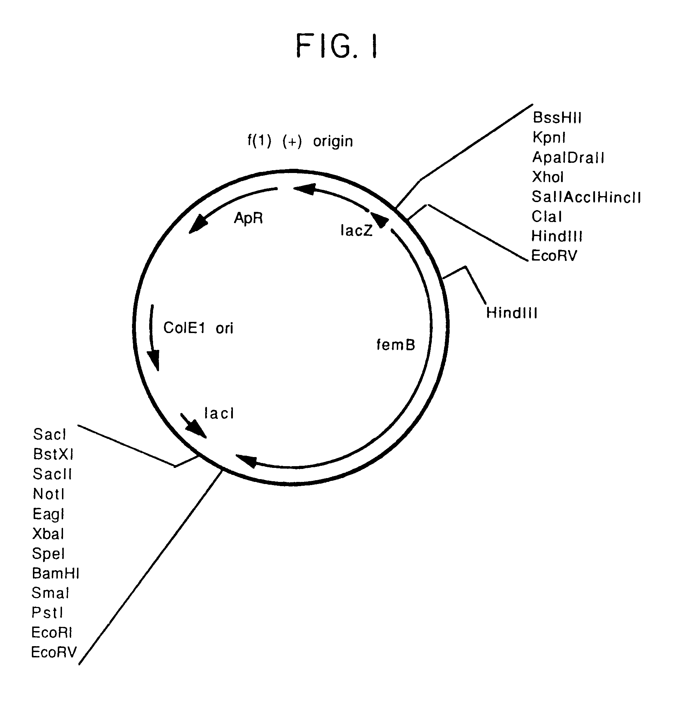

Synthesis of the FemB protein can be achieved by recombinant DNA technology. Synthetic genes, the *in vitro* or *in vivo* transcription and translation of which will result in the production of the FemB protein may be constructed by techniques well known in the art. Owing to the natural degeneracy of the genetic code, the skilled artisan will recognize that a sizable yet definite number of DNA sequences may be constructed which encode the FemB protein. All such genes are provided by the present invention. A preferred gene encoding the FemB protein is the natural *femB* gene of *Staphylococcus epidermidis,* which is SEQ ID NO: 1. This preferred *femB* gene is available on an ∼ 2.3 kb *EcoRV* restriction fragment of plasmid pPSJ203, publicly available and on deposit in *Escherichia coli* RR1 at the National Center for Agricultural Utilization Research, 1815 North University Street, Peoria, Illinois 61604-39999, under accession number NRRL B-21074 (date of deposit: April 7, 1993). A restriction site and function map of pPSJ203 is provided in Figure 1 of the drawings.

The *femB* gene may be created by synthetic methodology. Such methodology of synthetic gene construction is well known in the art. *See* Brown et al. (1979) *Methods in Enzymology,* Academic Press, N.Y., **68:**109-151. The *femB* DNA sequence may be generated using a conventional DNA synthesizing apparatus such as the Applied Biosystems Model 380A or 380B DNA synthesizers (commercially available from Applied Biosystems, Inc., 850 Lincoln Center Drive, Foster City, CA 94404).

To effect the translation of the FemB protein, one inserts the engineered synthetic DNA sequence in any of a large number of appropriate recombinant DNA expression vectors through the use of appropriate restriction endonucleases and DNA ligase. The synthetic *femB* gene should be designed to possess restriction endonuclease cleavage sites at either end of the transcript to facilitate isolation from and integration into these amplification and expression plasmids. The particular endonucleases employed will be dictated by the restriction endonuclease cleavage pattern of the parent expression vector to be employed. The choice of restriction sites are chosen so as to properly orient the FemB coding sequence with control sequences to achieve proper in-frame reading and expression of the FemB molecule. The FemB coding sequence must be positioned so as to be in proper reading frame with the promoter and ribosome binding site of the expression vector, both of which are functional in the host cell in which the FemB protein is to be expressed. The FemB protein may be expressed in any number of well-known eucaryotic or procaryotic hosts using known promoters and vectors. Some of the potential hosts, in addition to *E. coli,* include the yeasts *Saccharomyces cerevisiae* and *Pichia pastoris, Bacillus,* and cells infected with baculovirus.

To achieve efficient transcription of the synthetic gene, said gene must be operably associated with a promoter operator region. In one practice of the invention, the promoter-operator region of the synthetic gene encoding SEQ ID NO: 2 is placed in the same sequential orientation with respect to the ATG start codon of the synthetic gene as the promoter-operator occupies with respect to the ATG-start codon of the gene from which it was derived. Synthetic or modified promoter operator regions have been created and are well known in the art. When employing such synthetic or modified promoter-operator regions they should be oriented with respect to the ATG-start codon of the *femB* gene as directed by their creators. In one practice of the invention as exemplified herein, where the host cell is an *E. Coli* host cell, said promoter-operator region is the phage T7 promoter-operator region.

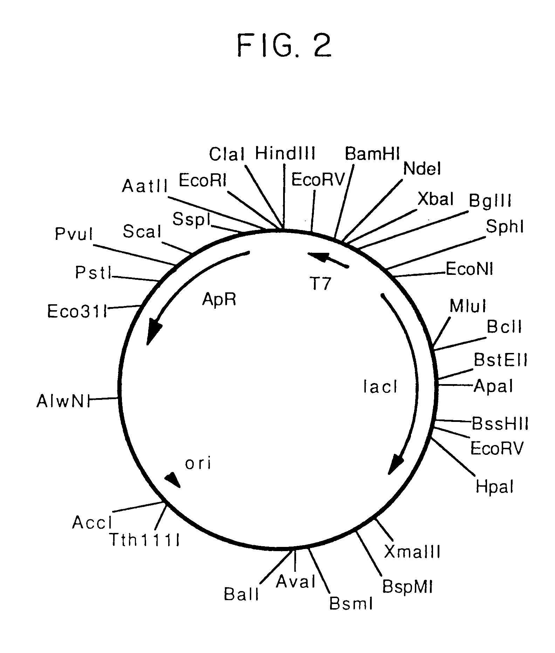

A variety of expression vectors useful for transforming procaryotic cells are well known in the art. A preferred vector for expression in an *E. coli* host cell is derived from *E. coli* plasmid pET-11A, which comprises the phage T7 promoter. A restriction site and function map of pET-11A appears in Figure 2 of the accompanying drawings. Plasmid pET-11A is publicly available from Novagen, Inc. (565 Science Drive, Madison, WI 53711) under catalog #69436-1. The preferred host strain is *E. coli* BL21(DE3), also available from Novagen under catalog #69387-1.

The techniques of transforming cells with the aforementioned vectors are well known in the art and may be found in such general references as Sambrook et al., *Molecular Cloning: A Laboratory Manual* (1988), Cold Spring Harbor Laboratory Press, Cold Spring Harbor, NY or Ausubel et al., *Current Protocols in Molecular Biology* (1989), John Wiley & Sons, New York, NY and supplements. The techniques involved in the transformation of *E. coli* cells used in the preferred practice of the invention as exemplified herein are well known in the art. The precise conditions under which the transformed *E. coli* cells are cultured is dependent on the nature of the *E. coli* host cell line and the expression or cloning vectors employed. For example, vectors which incorporate thermoinducible promoter-operator regions, such as the cI857 thermoinducible lambda-phage promoter-operator region, require a temperature shift from about 30 to about 40 °C. in the culture conditions so as to induce protein synthesis.

In a preferred embodiment of the invention *E. coli* K12 BL21 (DE3) cells were employed as host cells but numerous other cell lines are available. The transformed host cells are then plated on appropriate media under the selective pressure of the antibiotic corresponding to the resistance gene present on the expression plasmid. The cultures are then incubated for a time and temperature appropriate to the host cell line employed. Specifically, with *E. coli* K12 BL21 (DE3) cells, the *femB* gene is placed under the control of a promoter transcribed specifically by the T7 RNA polymerase. Induction of transcription of the *femB* gene is accomplished by the addition of isopropylthiogalactoside (IPTG) to the growth medium, which induces expression of the T7 RNA polymerase gene under the control of the *lacUV5* promoter. The T7 RNA polymerase is then available to transcribe the *femB* gene.

General techniques of protein purification are well-known to those of ordinary skill in the art. *See* Creighton, T.E., *Protein Structure: A Practical Approach* (1989), IRL Press, Oxford, England and Bollag, D.M. and Edelstein, S.J., *Protein Methods* (1991), Wiley-Liss, New York, NY. Proteins which are expressed in high-level bacterial expression systems characteristically aggregate in granules or inclusion bodies which contain high levels of the overexpressed protein. Kreuger et al. (1990) in *Protein Folding,* Gierasch and King, eds., pgs 136-142, American Association for the Advancement of Science Publication No. 89-18S, Washington, D.C. and Sambrook et al., *Molecular Cloning: A Laboratory Manual* (1988), pp. 17.37-17.41, Cold Spring Harbor Laboratory Press, Cold Spring Harbor, NY. The FemB protein sometimes aggregates into inclusion bodies when expressed under the control of phage T7 promoter. Such protein aggregates must be solubilized to provide further purification and isolation of the desired protein product. A variety of techniques using strongly denaturing solutions such as guanidinium-HCl and/or weakly denaturing solutions such as dithiothreitol (DTT) are used to solubilize the proteins.

Recombinantly produced proteins may be purified by a variety of techniques well known in the art such as ion exchange chromatography, size exclusion chromatography, electrophoresis, differential centrifugation, reversed phase high performance liquid chromatography, immunoaffinity chromatography, and the like. Protocols for use of these individual techniques or combinations thereof are well known in the art. Gradual removal of the denaturing agents (often by dialysis) in a refolding solution allows the denatured protein to assume its native conformation. The particular conditions for denaturation and refolding are determined by the particular protein expression system and/or protein in question. The S-sulfonates of the peptide molecules are converted to the disulfide paired, folded FemB molecules using a combination of high pH and added thiol in substantial accordance with the teaching of Frank, B.H. et al., (1981) in *Peptides. Synthesis, Structure and Function. Proceedings of the Seventh American* *Peptide Symposium* (Rich, D.H. and Gross, E., eds.) pp. 729-738, Pierce Chemical Co., Rockford, IL.

The *femB* gene may be used in gene disruption studies in *Staphylococcus epidermidis.* Although it is believed that the FemB protein is involved in the formation of a pentaglycine bridge in the cell wall of the bacterium, gene disruption will allow one to ascertain the precise effect of the loss of the *femB* gene. Gene disruption experiments in *Staphylococcus aureus* have revealed that a loss of *femB* results in an ∼40% reduction in cell wall glycine content. A similar result might be anticipated for *S*. *epidermidis.* Once determined, this information can be used to generate an assay for agents which inhibit the FemB protein, and are therefore useful in combination with antibiotics to treat methicillin-resistant bacteria.

The FemB protein of SEQ ID NO: 2 may be produced by recombinant methods. Recombinant methods are preferred if a high yield is desired. The present invention thus comprises a method for constructing a recombinant host cell capable of expressing SEQ ID NO: 2, said method comprising transforming a host cell with a recombinant DNA vector that comprises an isolated DNA sequence of Claim 1. The present invention also comprises a method for expressing SEQ ID NO: 2 in a recombinant host cell; said method comprising culturing said transformed host cell of Claim 5 under conditions suitable for gene expression.

The following Examples are provided to further illustrate and exemplify, but not limit the scope of, the invention.

### Example 1 Source of the Staphylococcus epidermidis femB gene

### Isolation of Plasmid PPSJ203

A lyophil of *E. coli* K12 RRl/pPSJ203 can be obtained from the Northern Regional Research Laboratories (NRRL), Peoria, Illinois 61604, under the accession number NRRL B-21074 (date of deposit: April 7, 1993). The pPSJ203 plasmid may be isolated from *E. coli* K12 RR1/pPSJ203 using techniques well-known to those skilled in the art. *See* Sambrook et al., *Molecular Cloning: A Laboratory Manual* (1988), Cold Spring Harbor Laboratory Press, Cold Spring Harbor, NY or Ausubel et al., *Current Protocols in Molecular Biology* (1989), John Wiley & Sons, New York, NY and supplements

### Isolation of the Staphylococcus epidermidis femB gene via the polymerase chain reaction

Isolated plasmid pPSJ203 is used as the template for the polymerase chain reaction at a concentration of 10 ng/reaction. Vent_{R}™ DNA polymerase (2 units/µl, Catalog #254, New England Biolabs, 32 Tozer Road, Beverly, MA 01915-9965) is used with standard Vent_{R}™ DNA polymerase buffer (1X= 10 mM KCl, 20 mM Tris-HCl (pH 8.8 at 25°C), 10 mM (NH₄)₂SO₄, 2 mM MgSO₄, 0.1% Triton X-100). The PCR primers used are GAAGGGAATTATCTAGATATGAAATTTACA (SEQ ID NO: 3) and ATCATTTATTTTTACGAATTCAATTTATTACGTATG (SEQ ID NO: 4). The reaction is carried out by 3 cycles of 94°C for 15 seconds, 40°C for 15 seconds and 72°C for 1 minute followed by 20 cycles of 94°C for 15 seconds, 55°C for 15 seconds and 72°c for 1 minute.

The reaction is transferred to a Centricon 100 microconcentrator (Amicon, Inc., 72 Cherry Hill, Beverly, MA 01915) and washed with 1 ml of water. The microconcentrator is then subjected to centrifugation at 3000 rpm in a microcentrifuge for 30 minutes. The reaction is then diluted to 200 µl (from ∼50 µl) with 1X *XbaI* restriction enzyme buffer. To this is added 50 units of *XbaI* and 50 units of *EcoRI.* The DNA is then digested for 90 minutes at 37°C. The DNA is phenol extracted and ethanol precipitated.

### Example 2 Construction of an Expression Plasmid Containing the femB Gene

The DNA created in Example 1 is then ligated to the 5.6 kb *XbaI-EcoRI* fragment of pET-11A (available from Novagen, Inc. (565 Science Drive, Madison, WI 53711) under catalog #69436-1. This plasmid is then transformed into *E. coli* BL21 (DE3) (also available from Novagen, Inc. under catalog #69387-1) using techniques well known to those of ordinary skill in the art.

### Example 3

### Expression of the FemB Protein

*E.* coli BL21 (DE3) transformed with the *femB* expression plasmid are grown overnight in TY broth (per liter 10 g tryptone, 5 g yeast extract and 5 g NaCl) and 100 µg/ml ampicillin. The cells are then diluted 1/50 into TY broth + ampicillin and grown at 37°C for 60 minutes. Expression is induced by adding ispropylthiogalactoside (IPTG) to 0.4 mM. Samples are taken at 0 and 6 hours and run on a % SDS-polyacrylamide gel using techniques described in Sambrook et al., *Molecular Cloning: A Laboratory Manual* (1988), pp. 18.47-18.59, Cold Spring Harbor Laboratory Press, Cold Spring Harbor, NY. An induced protein band is visible by staining with Coomassie Blue at the predicted size of about 51,000 daltons.

## Claims

1. A DNA compound that comprises an isolated DNA sequence encoding SEQ ID NO: 2.

2. The DNA compound of Claim 1 which comprises the isolated DNA sequence which is SEQ ID NO: 1.

3. A recombinant DNA vector that comprises the isolated DNA sequence of Claim 1.

4. A recombinant DNA vector of Claim 3 that further comprises a promoter positioned to drive expression of said isolated DNA sequence.

5. A method for constructing a recombinant host cell capable of expressing SEQ ID NO: 2, said method comprising transforming a host cell with a recombinant DNA vector that comprises an isolated DNA sequence of Claim 1.

6. A method for expressing SEQ ID NO: 2 in a recombinant host cell; said method comprising culturing said transformed host cell of Claim 5 under conditions suitable for gene expression.

7. A recombinant host cell transformed with a recombinant DNA vector of Claim 3.

8. The protein, in purified form, encoded by SEQ ID NO:2.
